# EUROPEAN PATENT SPECIFICATION

(11) **EP 1 230 551 B1**
(45) Date of publication and mention of the grant of the patent: **07.12.2005**
(21) Application number: 00969145.2
(22) Date of filing: 13.10.2000
(51) Int. Cl.: G01N 33/68, C07K 14/705

(54) **METHODS TO IDENTIFY COMPOUNDS THAT MODULATE NEURONAL ACTIVITY**
VERFAHREN ZUR IDENTIFIZIERUNG VON VERBINDUNGEN, DIE DIE NEURONALE AKTIVITÄT MODULIEREN
PROCEDE PERMETTANT D'IDENTIFIER DES COMPOSANTS MODULANT L'ACTIVITE NEURONALE

(30) Priority: 13.10.1999 US 159095 P
(43) Date of publication of application: 14.08.2002
(73) Proprietor: THE UNIVERSITY OF BRITISH COLUMBIA, Vancouver, British Columbia V6T 1Z3 (CA)
(72) Inventor: SNUTCH, Terrance, P., Vancouver, British Columbia V6S 1Z3 (CA)
(74) Representative: Fisher, Adrian John
(86) International application number: PCT/CA2000/001233
(87) International publication number: WO 2001/027630

(56) References cited:
- WO-A-95/04822
- WO-A-96/15149
- TU J C ET AL: "HOMER BINDS A NOVEL PROLINE-RICH MOTIF AND LINKS GROUP 1 METABOTROPIC GLUTAMATE RECEPTORS WITH IP3 RECEPTORS" NEURON,US,CAMBRIDGE, MA, vol. 21, October 1998 (1998-10), pages 717-726, XP000857914 cited in the application
- XIAIO B ET AL: "HOMER REGULATES THE ASSOCIATION OF GROUP 1 METABOTROPIC GLUTAMATE RECEPTORS WITH MULTIVALENT COMPLEXES OF HOMER-RELATED, SYNAPTIC PROTEINS" NEURON,US,CAMBRIDGE, MA, vol. 21, October 1998 (1998-10), pages 707-716, XP000857915 cited in the application
- ZHUCHENKO O ET AL: "AUTOSOMAL DOMINANT CEREBELLAR ATAXIA (SCA6) ASSOCIATED WITH SMALL POLYGLUTAMINE EXPANSIONS IN THE ALPHA1A-VOLTAGE-DEPENDENT CALCIUM CHANNEL" NATURE GENETICS,US,NEW YORK, NY, vol. 15, 7 January 1997 (1997-01-07), pages 62-69, XP002912380 ISSN: 1061-4036
- DATABASE WPI Section Ch, Week 199806 Derwent Publications Ltd., London, GB; Class B04, AN 1998-056555 XP002167930 & JP 09 299092 A (TAKEDA CHEM IND LTD), 25 November 1997 (1997-11-25)

## Description

### Technical Field

The invention relates to the field of neural transmission and drug discovery.

### Background Art

U.S. patents 6,090,631 and 5,623,051 describe a region of the P/Q and N-type calcium ion channels which binds syntaxin and SNAP-25 proteins. This region is located between domains II and III of the α₁ subunit of these channels. Syntaxin and SNAP-25 are proteins which mediate the docking of presynaptic vesicles required for release of neurotransmitters. As described in these documents, these syntaxin/SNAP-25 binding regions can be used to screen for compounds that will block the binding of these regions to syntaxin and/or SNAP-25, thus inhibiting neuronal transmission.

The conditions surrounding the transmission of signals through neuronal networks clearly affects a variety of physiological responses, including perception of pain, learning, memory, and the like. Modulation of the level of neural transmission and the condition of the presynaptic environment have profound physiological effects, primarily within the nervous system. The primary calcium ion channels that effect neural transmission are these N and P/Q type channels. P/Q type channels have been implicated mostly in the presynaptic terminals of the central nervous system (CNS) while the N type channels appear to dominate in the peripheral nervous system. Thus, P/Q type channels are particularly important in CNS functions such as memory and pain.

Calcium ion channels in general are composed of α₁ subunits which α₁ subunits are optionally coupled with additional subunits, but can function alone.

According to current terminology, N type channels are comprised of α_{1B} subunits while P/Q channels are composed of α_{1A} subunits. DNA sequences encoding α_{1A} subunits are described in WO 95 04822 and in Zhuchenko, O., *et al.* (1997) Nature Genetics 15:62-68.

It would clearly be desirable to provide compounds that are able to control the presynaptic environment so as to permit a greater control over central nervous system functions, including memory, learning and pain. The present invention provides a mechanism for this control. As will be shown below, a specific region of the α_{1A} subunit contains a sequence which binds to the known protein Homer which is described in articles by Xiao, B., *et al., Cur. Opinion in Neurobiol.* (2000) 10:370-374 and by Tu, J.C., *et al., Neuron* (1998) 21:717-726. As shown in these articles, the Homer protein binds to a multiplicity of targets which are important in signaling and neurotransmission. A consensus sequence which is proline rich is also described.

### Disclosure of the Invention

The invention resides in the identification of a peptide region specific to the P/Q calcium ion channel that is responsible for the cascade of events that results in expression of the gene encoding syntaxin-1A. Thus, use of this peptide in screening assays permits identification of compounds that can be used to regulate the levels of syntaxin-1A available in the presynaptic region and thus modulate such functions as learning, memory and pain.

According to the discovery of the applicants herein, calcium flow through the P/Q ion channel specifically effects the expression of the gene encoding syntaxin in model cell systems and in neuronal cells *per se.* It has now been found that a specific 4-amino acid sequence approximately 200 amino acids from the C terminus of the P/Q calcium ion channel is the site for interaction of this channel with Homer, a protein known to affect intracellular calcium ion stores, and this interaction is essential for the ability of the P/Q calcium ion channel to effect the expression of the syntaxin-1A encoding gene.

Thus, in one aspect, the invention is directed to a method to identify compounds that affect central nervous system function, such as learning and memory, which method comprises contacting a candidate compound with a peptide that comprises the binding site for Homer which resides proximal to the carboxy terminus of the P/Q calcium ion channel and determining whether said compound binds to said peptide. Compounds that bind to this peptide are identified as compounds that affect central nervous system (CNS) function. This screening assay can be conducted in a straightforward manner by simply assessing the ability of the compound to bind. More commonly, the ability of the candidate compound to inhibit the binding of a ligand known to bind the peptide, including the ability of Homer to so bind, can be used to assess said binding.

In another aspect, the invention is directed to a peptide comprising the sequence of the binding site flanked by additional amino acids, typically those which flank the binding site in the native ion channel, and to antibiotics that are immunospecific for this region.

### Modes of Carrying Out the Invention

The present applicants have shown that calcium influx selectively through P/Q type calcium ion channels is responsible for activating expression of syntaxin-1A, a presynaptic protein that plays a central role in mediating vesicle docking, fusion and neurotransmitter release. Applicants have demonstrated that the initial calcium ion signal is amplified through calcium ion from intracellular stores and acts via phosphorylation that is dependent on a number of co factors including CaMK II/IV, PKA, and MAPKK. As syntaxin-1A interacts with P/Q type calcium ion channels to decrease channel availability, the expression of the gene encoding syntaxin-1A is regulated by an activity-dependent feedback pathway.

Applicants have now shown that the interaction of the initial extracellular calcium signal with the calcium released from intracellular stores is mediated by binding of the known protein Homer to a specific amino acid sequence proximal to the C terminus of the P/Q type ion channel and is specific for the P/Q α_{1A} subunit as opposed to other known calcium ion channels. Identification of this binding site permits the use of peptides containing this site as screening tools for important compounds which modulate CNS activity.

Methods for conducting such screening assays are well known in the art. Typically, the target peptide is produced recombinantly in suitable host cells and displayed at the surface of said host cells or is coupled to a solid support. Such coupling may be covalent coupling or may be achieved through non-covalent adsorption, such as for example, by providing a histidine tag and associating the resulting fusion protein to a solid support through a metal chelate. A wide variety of methods for providing an assayable form of the peptide is known in the art. Indeed, as the required peptide is relatively short, direct synthesis on a solid support is one convenient way of providing this peptide.

The specific site required for binding is the four amino acid sequence PLMF. In order to render the assay practical and specific, however, it is desirable to include additional amino acid sequence at one or both of the N and C terminus of this tetrapeptide. Preferred embodiments for such additional amino acid sequence are the sequences present in the native ion channels. Typically, sequences extending 20 amino acids upstream and/or downstream, more preferably 15 amino acids upstream and/or downstream, or even 10 or 5 or 2 amino acids of the sequences upstream and/or downstream of the required tetrapeptide are employed.

The sequence containing the tetrapeptide with the tetrapeptide itself in bolded and underlined types is as follows:
1966-YYRQSKAKKL QAMREEQDRT **PLMF**QRMEPP SPTQEGGPGQ NALP-2009

This sequence is derived from one allele encoding a human P/Q type calcium channel. It is understood that similar sequences are included in the corresponding P/Q channels of other mammalian species, and peptides derived from these species as well as from allelic variants of the sequence shown above may be used in the method of the invention. In general, it is preferred to utilize peptides containing sequence derived from the above-shown sequence which are at least 90% homologous, preferably 95% homologous, more preferably 97% homologous and more preferably 99% homologous with the sequence shown above, or a fragment thereof as long the required tetrapeptide is included.

Methods for conducting the assay once the peptide is made available, preferably displayed on cells or on a solid support, are well known in the art. As noted above, compounds can be assessed for binding directly by providing them with labels, or, for example, using homogenous assays wherein the peptide itself is labeled and the detectability of signal ascribed to the label is affected by the peptide being in a bound or unbound state. Alternatively, and perhaps more conveniently, the binding may be assessed by ability of the compound to inhibit the binding of a ligand known to bind to the P/Q peptide, and to the tetrapeptide essential portion thereof specifically. Such "ligands" would include antibodies specific to this epitope, including antibodies in the various forms, such as single-chain Fv regions, Fab fragments, etc., the Homer protein which is the native ligand, or, indeed, any compound which has previously been determined to bind to this site. A wide variety of detection methods for such inhibition of known ligand binding is known. Typically, the competing known ligand is labeled and a decrease in label bound to the solid support (if the peptide is thus supported) provides an assessment of such inhibition. Labels may include fluorescent labels, chromogenic labels, enzyme labels, radioisotope labels, and the like. Again, homogenous assays may be convenient. For example, fluorescence quenching assays wherein both the peptide and the competing ligand contain label are known. The invention resides in the nature of the peptide used as the target, not in the specific design of the screening assay.

Compounds that are identified in this screen will clearly influence the status of signaling in the neuronal pathway of a subject administered these compounds. The subject may be a laboratory animal used to study patterns of learning and memory, such as rats, mice and rabbits. The subject may also be a human subject where it is desired to alter a deficit in memory or assuage some other physiological disorder. Administration of such compounds to subjects is by standard procedures and uses formulations such as those in Remington's Pharmaceutical Sciences, latest edition, Mack Publishing Co., Easton, PA. Thus, the compounds identified using the method of the invention could, for example, be administered to murine models of specific CNS based or other neurological based conditions and the effect of such compounds on the model will elucidate the mechanisms associated with the condition.

The applicants herein have verified the nature of the elevation of expression of syntaxin-1A through calcium ion transit of the ion channel P/Q as follows:

First, it was shown that transient expression of human α_{1A} (P/Q type) calcium channels in HEK293 cells resulted in a negative steady state inactivation which, in turn, suggested a corresponding interaction with syntaxin. This was confirmed by incubation with botulism toxin Cl which shifted the steady state inactivation, but did not affect the voltage dependence of current activation. In addition, these results were affected as expected by the presence of an antisense syntaxin construct.

It was further shown that syntaxin-1A was not endogenously expressed in HEK293 cells while the presence of the syntaxin-1A protein was detected in HEK293 cells transfected with the α_{1A} P/Q type calcium channel; mRNA corresponding to syntaxin-1A was also detected, but inhibited when the cells were incubated in actinomycin D. Syntaxin-1A was the only SNARE protein produced in these cells; syntaxin 1B, SNAP-25, synaptophysin, VAMP, and synaptotagmin were not detected.

There was no evidence for production of syntaxin-1A in HEK293 cells transfected with calcium ion channel α₂δ or β_{1B} subunits. Cells transfected with α_{1B} (N type); α_{1C} (L type); α_{1E} (novel type); and the α_{1G} and a₁₁ T type channels also failed to result in production of syntaxin-1A mRNA. The possibility that the specificity of P/Q type channels in inducing syntaxin-1A production was eliminated using appropriate controls.

In the presence of various selected P/Q type channel antagonists, production of syntaxin-1A, mRNA or protein was undetectable.

It was confirmed that the presence of the P Q type channels in transfected HEK293 cells resulted in an enhanced intracellular calcium ion concentration. It appears to be this intracellular enhanced calcium ion concentration which results in syntaxin-1A production since ionomycin at concentrations of 10 nM-2 µM, which non-specifically enhances intracellular calcium ion concentration, defined a discrete upper and lower limit of intracellular calcium ion dependent activation with maximal activation occurring at levels of 50-200 nM.

Having established that expression of the syntaxin-1A gene is specifically induced by calcium flux mediated by P/Q ion channels, applicants elucidated the subsequent transduction pathway using agents that alter intracellular calcium concentration. Application of thapsigargin stimulated syntaxin-1A expression in untransfected cells, and, in α_{1A} transfected cells both BAPTA-AM and EGTA-AM blocked the calcium dependent induction of syntaxin-1A. Either caffeine or carbachol activated calcium release from ryanodine - and IP₃ - sensitive stores rapidly and stimulated syntaxin-1A mRNA levels in untransfected cells. This was inhibited using agents which block release from either of these stores. In general, the foregoing data suggest that a discrete level of basil α_{1A} specific calcium influx induces syntaxin-1A expression through a secondary calcium release from intracellular stores. Syntaxin-1 A expression in α_{1A} transfected cells could be inhibited with compounds known to block tyrosine kinase, calmodulin activity or CaMK II/IV or PKA activities. Activators of PKA induced syntaxin mRNA even in the absence of α_{1A} transfection. Activation of protein kinase C did not induce expression. A portion of the transduction pathway may also involve the transcription factor CREB, as it was determined that the level of phosphorylated CREB is up-regulated in the transfected HEK cells.

The foregoing results in HEK293 transfected cells were correlated with the endogenous P/Q type channels in neurons. Syntaxin-1A is basally expressed in cerebellar granule cells, but this is inhibited by ω Agatoxin IVA, which is known to inhibit calcium P/Q type channels specifically. Inhibitors of N or L type channels did not block calcium dependent expression of syntaxin-1A. Treatment of these cerebellar granule cells, which had been incubated in ω Agatoxin IVA with thapsigargin recovered the syntaxin-1A expression.

Syntaxin-1A expression was inhibited by the same agents as had inhibited it in HEK293 cells including inhibitors of ryanodine - or IP₃-induced calcium release from stores, of CaM kinase II/IV, and of PKA, or MAPKK.

In summary, P/Q type selective calcium influx induces expression of syntaxin-1A but not of other SNARE proteins involved in vesicle fusion and neurotransmitter release. It appears to be under tight spatial and temporal calcium dependent control and is apparently mediated by an association with intracellular calcium stores.

Further, applicants have identified the initial critical step in the transduction pathway which is the binding of the Homer protein to a specific site in the α_{1A} channel subunit proximal to the C terminus. The availability of a peptide containing this site permits the identification of compounds that are useful in elucidating neuronal pathways, and in modulating the CNS in subjects in general, including treating disorders of the CNS especially those involving learning and memory.

## Claims

1. A method to identify compounds that affect learning and memory which method comprises
contacting a candidate compound with a peptide that comprises the binding site for Homer proximate to the carboxy terminus of a mammalian P/Q calcium ion channel;
wherein said peptide comprises the amino acid sequence PLMF and no more than 20 amino acids upstream and/or downstream of the sequence PLMF in said P/Q ion channel; or
wherein the peptide is at least 90% homologous to the peptide 1966-YYRQSKAKKL QAMREEQDRT PLMFQRMEPP SPTQEGGPGQ NALP-2009 or a fragment thereof, provided that said peptide comprises the sequence PLMF and no more than 20 amino acids upstream and/or downstream of said PLMF sequence; and
determining whether said compound binds to said peptide;
wherein a candidate compound which binds to said peptide is identified as a compound that affects learning or memory.

2. The method of claim 1 wherein said contacting is in the presence of a ligand known to bind said peptide, and said determining comprises assessing the ability of a candidate compound to displace said ligand.

3. The method of claim 1 wherein the PLMF sequence is flanked by 10 amino acids upstream and/or downstream of the sequence PLMF in said P/Q ion channel.

4. The method of claim 1 wherein the peptide is at least 95% homologous to the peptide 1966-YYRQSKAKKL QAMREEQDRT PLMFQRMEPP SPTQEGGPGQ NALP-2009 or a fragment thereof which comprises PLMF.

5. The method of any of claims 1-4 wherein the peptide is displayed on cells which contain a recombinant expression system which comprises a nucleotide sequence encoding said peptide operably linked to sequences that effect expression.

6. A peptide which comprises the amino acid sequence PLMF and no more than 20 amino acids upstream and/or downstream of the sequence PLMF in a P/Q ion channel; or a peptide at least 90% homologous to the peptide 1966-YYRQSKAKKL QAMREEQDRT PLMFQRMEPP SPTQEGGPGQ NALP-2009 or a fragment thereof, provided that said peptide comprises the sequence PLMF and no more than 20 amino acids upstream and/or downstream of said PLMF sequence.

7. The peptide of claim 6 wherein the sequence PLMF is flanked by sequences of 10 amino acids corresponding to those immediately upstream and downstream of the sequence PLMF in said P/Q calcium ion channel.

8. The peptide of claim 6 which is at least 95% homologous to the sequence 1966-YYRQSKAKKL QAMREEQDRT PLMFQRMEPP SPTQEGGPGQ NALP-2009 or a fragment thereof containing PLMF.

9. Antibodies specifically immunoreactive with the peptide of any of claims 6-8.

10. A recombinant expression system for a peptide of any of claims 6-8 which comprises a nucleotide sequence encoding said peptide operably linked to sequences that effect expression.

11. Cells which comprise the expression system of claim 10.

12. A method to produce a peptide comprising the binding site for Homer which method comprises culturing the cells of claim 11 under conditions wherein a peptide is produced.

13. Cells obtained by the method of claim 12 which display said peptide.

## Patentansprüche

1. Verfahren zum Identifizieren von Verbindungen, die Lernen und Gedächtnis beeinträchtigen, welches Verfahren umfasst:
Kontaktieren einer Kandidatenverbindung mit einem Peptid, welches die Bindungsstelle für ein Homer nahe dem Carboxyterminus des Säugetier P/Q-Kalziumionenkanals; umfasst;
worin das Peptid die Aminosäuresequenz PLMF und nicht mehr als 20 Aminosäuren stromaufwärts und/oder stromabwärts der Sequenz PLMF in dem P/Q-Ionenkanal umfasst; oder
worin das Peptid wenigstens 90% homolog zu dem Peptid 1966-YYRQSKAKKL QAMREEQDRT PLMFQRMEPP SPTQEGGPGQ NALP-2009 oder einem Fragment davon ist, vorausgesetzt, dass das Peptid die Sequenz PLMF umfasst und nicht mehr als 20 Aminosäuren stromaufwärts und/oder stromabwärts der PLMF-Sequenz; und
Bestimmen, ob die Sequenz an das Peptid bindet,
worin eine Kandidatenverbindung, welche an das Peptid bindet, als Verbindung identifiziert wird, die Lernen oder Gedächtnis beeinträchtigt.

2. Verfahren nach Anspruch 1, worin das Kontaktieren in Gegenwart eines Liganden geschieht, von dem bekannt ist, dass er an das Peptid bindet, und die Bestimmung die Einschätzung des Vermögens der Kandiatenverbindung umfasst, den Liganden zu ersetzen.

3. Verfahren nach Anspruch 1, worin die PLMF-Sequenz flankiert wird durch 10 Aminosäuren stromaufwärts und/oder stromabwärts der PLMF-Sequenz des P/Q-lonenkanals.

4. Verfahren nach Anspruch 1, worin das Peptid wenigstens 95% homolog zu dem Peptid 1966-YYRQSKAKKL QAMREEQDRT PLMFQRMEPP SPTQEGGPGQ NALP-2009 ist oder von einem Fragment davon, welches PLMF umfasst.

5. Verfahren nach einem der Ansprüche 1 bis 4, worin das Peptid auf Zellen gezeigt wird, welche ein rekombinantes Expressionssystem enthalten, das eine Nukleotidsequenz umfasst, die das Peptid kodiert, betreibbar verknüpft an Sequenzen, welche die Expression bewirken.

6. Peptid, welches die Aminosäuresequenz PLMF und nicht mehr als 20 Aminosäuren stromaufwärts und/oder stromabwärts der Sequenz PLMF in einem P/Q-Ionenkanal umfasst oder ein Peptid, das wenigstens 90% homolog zu dem Peptid 1966-YYRQSKAKKL QAMREEQDRT PLMFQRMEPP SPTQEGGPGQ NALP-2009 oder einem Fragment davon ist, vorausgesetzt, dass das Peptid die Sequenz PLMF und nicht mehr als 20 Aminosäuren stromaufwärts und/oder stromabwärts der PLMF-Sequenz umfasst.

7. Peptid nach Anspruch 6, worin die PLMF-Sequenz flankiert wird durch 10 Aminosäuren stromaufwärts und/oder stromabwärts der PLMF-Sequenz des P/Q-Ionenkanals.

8. Peptid nach Anspruch 6, welches wenigstens 95% homolog zur Sequenz 1966-YYRQSKAKKL QAMREEQDRT PLMFQRMEPP SPTQEGGPGQ NALP-2009 ist oder von einem Fragment davon, welches PLMF enthält.

9. Antikörper, die spezifisch immunoreaktiv mit dem Peptid nach einem der Ansprüche 6 bis 8 sind.

10. Rekombinantes Expressionssystem für ein Peptid nach einem der Ansprüche 6 bis 8, welches eine Nukleotidsequenz umfasst, die das Peptid kodiert, betreibbar verknüpft an Sequenzen, die die Expression bewirken.

11. Zellen, welche das Expressionssystem nach Anspruch 10 umfassen.

12. Verfahren zum Herstellen eines Peptids, das die Bindungsstelle für ein Homer umfasst, welches Verfahren das Kultivieren der Zellen nach Anspruch 11 unter Bedingungen umfasst, wo ein Peptid erzeugt wird.

13. Zellen, erhalten durch das Verfahren nach Anspruch 12, welche das Peptid zeigen.

## Revendications

1. Procédé d'identification de composés qui affectent l'apprentissage et la mémoire, ce procédé comprenant
la mise d'un composé candidat en contact avec un peptide qui comprend le site de liaison pour Homer proche de la terminaison carboxy d'un canal d'ions calcium P/Q de mammifère ;
dans lequel le peptide comprend la séquence d'acides aminés PLMF et pas plus de 20 acides aminés en amont et/ou en aval de la séquence PLMF du canal d'ions P/Q ; ou
dans lequel le peptide est homologue à au moins 90 % du peptide 1966-YYRQSKAKKL QAMREEQDRT PLMFQRMEPP SPTQEGGPGQ NALP-2009 ou de l'un de leurs fragments, pourvu que le peptide comprenne la séquence PLMF et pas plus de 20 acides aminés en amont et/ou en aval de la séquence PLMF ; et
la détermination du point de savoir si le composé se fixe au peptide ;
dans lequel un composé candidat qui se fixe au peptide est identifié comme un composé qui affecte l'apprentissage ou la mémoire.

2. Procédé suivant la revendication 1, dans lequel on effectue la mise en contact en la présence d'un ligand connu pour fixer le peptide, et la détermination comprend l'estimation de l'aptitude d'un composé candidat à déplacer le ligand.

3. Procédé suivant la revendication 1, dans lequel la séquence PLMF est flanquée de 10 acides aminés en amont et/ou en aval de la séquence PLMF du canal d'ions P/Q.

4. Procédé suivant la revendication 1, dans lequel le peptide est homologue à au moins 95 % du peptide 1966-YYRQSKAKKL QAMREEQDRT PLMFQRMEPP SPTQEGGPGQ NALP-2009 ou de l'un de leurs fragments qui comprend PLMF.

5. Procédé suivant l'une quelconque des revendications 1 à 4, dans lequel le peptide est présent sur des cellules qui contiennent un système d'expression recombinant qui comprend une séquence de nucléotides codant le peptide lié fonctionnellement à des séquences qui effectuent une expression.

6. Peptide qui comprend la séquence d'acides aminés PLMF et pas plus de 20 acides aminés en amont et/ou en aval de la séquence PLMF d'un canal d'ions P/Q ; ou un peptide homologue à au moins 90 % du peptide 1966-YYRQSKAKKL QAMREEQDRT PLMFQRMEPP SPTQEGGPGQ NALP-2009 ou de l'un de leurs fragments, pourvu que le peptide comprenne la séquence PLMF et pas plus de 20 acides aminés en amont et/ou en aval de la séquence PLMF.

7. Peptide suivant la revendication 6, dans lequel la séquence PLMF est flanquée de séquences de 10 acides aminés correspondant à ceux immédiatement en amont et en aval de la séquence PLMF du canal d'ions calcium P/Q.

8. Peptide suivant la revendication 6 qui est homologue à au moins 95 % de la séquence 1966-YYRQSKAKKL QAMREEQDRT PLMFQRMEPP SPTQEGGPGQ NALP-2009 ou de l'un de leurs fragments contenant PLMF.

9. Anticorps spécifiquement immunoréactifs avec le peptide de l'une quelconque des revendications 6 à 8.

10. Système d'expression recombinant pour un peptide suivant l'une quelconque des revendications 6 à 8, qui comprend une séquence de nucléotides codant le peptide lié fonctionnellement à des séquences qui effectuent une expression.

11. Cellules qui comprennent le système d'expression de la revendication 10.

12. Procédé de production d'un peptide comprenant le site de liaison pour Homer, ce procédé comprenant la culture des cellules de la revendication 11 dans des conditions dans lesquelles un peptide est produit.

13. Cellules obtenues par le procédé de la revendication 12, qui présentent le peptide.


### SEQUENCE LISTING

<110> NeuroMed Technologies, Inc.
<120> METHODS TO IDENTIFY COMPOUNDS THAT MODULATE NEURONAL ACTIVITY
<130> 49324-9
<140> PCT/CA00/01233
   <141> 2000-10-13
<150> 60/159,095
   <151> 1999-10-13
<160> 1
<170> FastSEQ for Windows Version 4.0
<210> 1
   <211> 44
   <212> PRT
   <213> Homo sapiens
<400> 1
   Tyr Tyr Arg Gln Ser Lys Ala Lys Lys Leu Gln Ala Met Arg Glu Glu
    1               5                  10                  15
   Gln Asp Arg Thr Pro Leu Met Phe Gln Arg Met Glu Pro Pro Ser Pro
               20                  25                  30
   Thr Gln Glu Gly Gly Pro Gly Gln Asn Ala Leu Pro
           35                  40